# EUROPÄISCHE PATENTSCHRIFT

(11) **EP 0 791 574 B1**
(45) Veröffentlichungstag und Bekanntmachung des Hinweises auf die Patenterteilung: **03.05.2000**
(21) Anmeldenummer: 97102053.2
(22) Anmeldetag: 10.02.1997
(51) Int. Cl.: C07C 68/02, C07C 69/96

(54) **Verfahren zur kontinuierlichen Herstellung von Arylcarbonaten**
Process for the continuous preparation of aryl carbonates
Procédé de fabrication continue de carbonates d'aryle

(30) Priorität: 21.02.1996 DE 19606384
(43) Veröffentlichungstag der Anmeldung: 27.08.1997
(73) Patentinhaber: BAYER AG, 51368 Leverkusen (DE)
(72) Erfinder: Ooms, Pieter, Dr., 47800 Krefeld (DE); Buysch, Hans-Josef, Dr., 47809 Krefeld (DE); Kühling, Steffen, Dr., 40670 Meerbusch (DE); Zaby, Gottfried, Dr., 51375 Leverkusen (DE)

(56) Entgegenhaltungen:
- EP-A- 0 757 029

## Beschreibung

Die vorliegende Erfindung betrifft ein Verfahren zur kontinuierlichen Herstellung von Carbonaten mit aromatischen Estergruppen durch Umsetzung von aromatischen Hydroxyverbindungen mit Phosgen in Gegenwart von heterogenen Katalysatoren, wobei durch Rückführung des Abgases in einen Nachreaktor noch vorhandenes Phosgen aus dem Abgasstrom entfernt wird.

Es ist bekannt, daß Arylcarbonate durch Phasengrenzflächenphosgenierung (Schotten-Baumann-Reaktion) von aromatischen Hydroxyverbindungen gewonnen werden können. Dabei wirkt sich die Verwendung von Lösungsmitteln und Natronlauge nachteilig aus, da durch die wässrige Lauge eine teilweise Verseifung von Phosgen oder Chlorkohlensäureester stattfinden kann, große Mengen Kochsalz als Nebenprodukt anfallen und das Lösungsmittel zurückgewonnen werden muß.

Vorschläge für Verfahren ohne Lösungsmittel finden sich z.B. in US-A 2 837 555; 3 234 263; 2 362 865. Es werden jedoch lösliche Katalysatoren verwendet, deren Abtrennung von den Produkten aufwendig ist.

Somit erscheint es sinnvoll, heterogene, nicht lösliche Katalysatoren zu verwenden, die eine Aufarbeitung des Reaktionsgemisches wesentlich erleichtern. Auch dazu wurden Vorschläge gemacht. So wird in der EP-A-516 355 vor allem Aluminiumtrifluorid empfohlen, das gegebenenfalls auf Träger wie Alumosilikate aufgebracht wird. Die Synthese von Aluminiumfluorid ist jedoch durch die Handhabung von Fluor oder Flußsäure sehr aufwendig und teurer. Weiter werden in der WO 91/06526 Metallsalze auf porösen Trägern als Katalysatoren für die erfindungsgemäßen Umsetzungen beschrieben. Eine vollkontinuierliche Phosgenierung von Phenol ist an solchen Katalysatoren nur in der Gasphase möglich, was aber relativ hohe Reaktionstemperaturen und die Gefahr der Zersetzung der empfindlichen Chlorameisensäureester mit sich bringt. Offensichtlich ist eine Phosgenierung von Phenol mit diesen Katalysatoren in der Flüssigphase nicht durchführbar, da das heiße, flüssige Phenol die aktiven Katalysatorbestandteile auswäscht.

Die EP-A-757 029 enthält einen Lösungsvorschlag eine kontinuierliche Verfahrensweise zur Herstellung von Diarylcarbonaten durch Phosgenierung von aromatischen Hydroxyverbindungen in Gegenwart von heterogenen Katalysatoren.

Als weitere Lösung wird nun ein Verfahren vorgeschlagen, bei dem man
1) ein Gemisch aus aromatischer Hydroxyverbindung und gegebenenfalls deren Chlorameisensäureester zusammen mit Phosgen in einen mit heterogenem Katalysator gefüllten Reaktor einleitet und darin so reagieren läßt, daß die Reaktionswärme durch Verdampfen der Edukte und Produkte abgeführt wird und so die Reaktionstemperatur um maximal 50°C über die Eintrittstemperatur des Reaktionsgemisches steigt,
2) das den Reaktor verlassende Produkt entgast, das Abgas zusammen mit einem gerade schmelzflüssigen Strom der aromatischen Hydroxyverbindung, der gegebenenfalls etwas Chlorameisensäurester enthält, in einen mit heterogenem Katalysator gefüllten Abgasnachreaktor einleitet und darin so reagieren läßt, daß Phosgen und gegebenenfalls Chlorameisensäureester aus dem Abgasstrom entfernt werden,
3) das aus dem Reaktor entnommene und entgaste Reaktionsprodukt entweder direkt der Aufarbeitung zuführt oder aber in einen Nachreaktor einspeist, in dem restlicher Chlorameisensäureester über heterogenem Katalysator mit noch vorhandender oder zugespeister aromatischer Hydroxyverbindung weiter zu Diarylcarbonat umgesetzt wird,
4) das den Nachreaktor verlassende Produkt wiederum entgast und dieses Abgas dem unter 2) genannten Abgasnachreaktor mit schmelzflüssiger aromatischer Hydroxyverbindung zuführt,
5) das entgaste Produkt aus dem Nachreaktor in eine Destillationskolonne einspeist, aromatische Hydroxyverbindung und gegebenenfalls noch vorhandene Spuren Chlorameisensäureester über Kopf abdestilliert und in den Abgasnachreaktor oder in den ersten Reaktor wieder einleitet,
6) den Sumpf dieser ersten Kolonne einer zweiten Destillationskolonne zuleitet, in ihr über Kopf gegebenenfalls noch vorhandene Spuren Leichtsieder aus dem Diarylcarbonat entfernt, die in den oberen Teil der ersten Kolonne zurückgegeben werden,
7) aus dem Gasraum der zweiten Kolonne Diarylcarbonat, gegebenenfalls mit Spuren Hochsieder, ausspeist,
8) dieses Produkt einer dritten Kolonne zuführt und durch Abtrennung von Restmengen Hochsieder als Sumpf reines Diphenylcarbonat als Kopfprodukt erhält,
9) die vereinigten Sümpfe der zweiten und dritten Kolonne einer vierten Destillationseinheit zuführt, über Kopf Diarylcarbonat abdestilliert, es in die zweite Kolonne zurückleitet und aus dem Sumpf der vierten Destillationseinheit die Hochsieder entnimmt.

Aromatische Hydroxyverbindungen für das erfindungsgemäße Verfahren sind solche der Formel

ArOH,

worin
- Ar: Phenyl, Naphthyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen und heterocyclischen Reste durch einen oder mehrere Substituenten wie geradkettige oder verzweigte C₁-C₄-Alkyl-, C₁-C₄-Alkenyl-, C₁-C₄-Alkoxygruppen, Phenylreste oder Nitril- und Halogenfunktionen substituiert sein können, und wobei weiterhin die heterocyclischen Reste mit einem ankondensierten Benzolkern verbunden sein können.

Beispiele für erfindungsgemäße, aromatische Hydroxyverbindungen sind: Phenol, o-, m- und p-Kresol, o-, m- und p-Isopropylphenol, die entsprechenden Halogen-bzw. Alkoxyphenole, wie p-Chlorphenol bzw. p-Methoxyphenol, ferner Monohydroxyverbindungen des Naphthalins, Anthracens und Phenanthrens. weiterhin 4-Hydroxypyridin und Hydroxychinoline. Vorzugsweise werden substituierte Phenole, ganz besonders bevorzugt Phenol selbst eingesetzt.

Geeignete Katalysatoren für das erfindungsgemäße Verfahren sind im Prinzip bekannt, beispielsweise aus EP-A-483 632, EP-A-635 476, US-A-5 478 961, EP-A-635 477, US-A-5 473 094, EP-A-645 364, EP-A-691 326, EP-A-516 355, US-A-5 239 105 und US-A-5 136 077.

Die Edukte Phosgen und Hydroxyverbindung setzt man in Molverhältnissen von 1:0,5 bis 1:8, bevorzugt 1:1,5 bis 1:5, besonders bevorzugt 1:2 bis 1:4 ein. Das stöchiometrische Verhältnis ist in diesem Fall 1:2.

Die Katalysatoren werden in der Regel als körniges Material, Granulate, Extrudate, Stäbchen, Kugeln, oberflächenreiche Formkörper wie Hohlextrudate in Form von Raschigringen, Hohlzylindern, Sternen, Wagenrädern oder als Bruchstücke eingesetzt. Durchmesser und Länge dieser Teilchen betragen 0,5 bis 10 mm. Sie werden im Reaktor in Form einfacher Schüttungen angeordnet.

Geeignete Reaktoren für das erfindungsgemäße Verfahren sind dem Fachmann bekannt. Beispiele sind Röhrenreaktoren, gegebenenfalls mit Kühl- bzw. Heizmantel, die den Katalysator als Schüttung enthalten, oder Hordenreaktoren, in denen der Katalysator als gleichmäßige Schicht auf mehrere übereinanderliegende Böden verteilt ist.

Phosgen und aromatische Hydroxyverbindung können zur Umsetzung im Gleich- oder im Gegenstrom durch den Reaktor geleitet werden. Bei senkrecht stehenden Reaktoren kann man die flüssige Phase sowohl von oben nach unten als auch von unten nach oben durch den Reaktor leiten.

Die Umsetzung von Phosgen und aromatischer Hydroxyverbindung wird durchgeführt bei Temperaturen von etwa 100 bis 250°C, vorzugsweise 120 bis 230°C, besonders bevorzugt 130 bis 220°C. Die Reaktionswärme wird durch Verdampfen von Edukten und Produkten soweit abgeführt, daß die Temperatur des Reaktionsgemisches um maximal 50°C, bevorzugt höchstens 40°C, besonders bevorzugt nicht mehr als 35°C, über die Eintrittstemperatur der Reaktanden steigt.

Der Druck bewegt sich im Bereich von 0,3 bis 10 bar, bevorzugt 0,5 bis 7 bar, besonders bevorzugt von 0,8 bis 6 bar.

Das bei der Reaktion gebildete Abgas wird durch einen mit heterogenem Katalysator gefüllten Reaktor mit einem gerade schmelzflüssigen Strom der Hydroxyverbindung, der auch Chlorameisensäureester in Mengen von <50 Gew.-%, vorzugsweise <30 Gew.- %, besonders bevorzugt <10 Gew.-% enthalten kann, in Gleich- oder Gegenstrom geleitet, wobei dem Gasstrom restliches Phosgen und gegebenenfalls noch mitgeführte kleinere Mengen an Chlorameisensäureester entzogen werden. Das den Abgasnachreaktor verlassende flüssige Gemisch wird nach Entgasung durch Zugabe von Phosgen und gegebenenfalls weiterer Hydroxyverbindung auf das gewünschte Molverhältnis eingestellt, auf die gewünschte Temperatur erhitzt und dem Reaktor zugeführt.

Der nach Entgasung des Reaktionsgemisches aus dem Abgasnachreaktor am Kopf austretende Gasstrom besteht im wesentlichen aus Chlorwasserstoff. Noch eventuell vorhandene Phosgenspuren können nach bekannten Methoden in einem A-Kohleturm mit wenig Wasser hydrolysiert werden. Die entsprechend ihrem Dampfdruck bei der in der Entgasungsapparatur herrschenden Temperatur noch im Chlorwasserstoffstrom befindliche Menge der Hydroxyverbindung wird durch Ausfrieren in einer Kühlfalle entfernt und kann in den Reaktor zurückgeführt werden. Eine eventuelle Restmenge wird in der nachfolgenden adiabatischen Adsorption des Chlorwasserstoffes in Wasser durch azeotrope Destillation als wäßrige Mischung ausgetrieben und kann nach Wiedergewinnung dem Reaktor zugeführt oder aber für andere Zwecke, wie der Herstellung von Phenolharzen verwendet werden.

Die restlichen Phosgenmengen, die sich noch im Chlorwasserstoff befinden. können auch vorteilhaft nach der adiabatischen Absorption mit Wasser, wo sie mit dem Azeotrop aus Hydroxyverbindung und Wasser ausgetrieben werden, mit den restlichen aus den Eduktströmen stammenden Inertgasspuren dem A-Kohleturm zugeführt und darin hydrolysiert werden.

Nach der Entgasung des Reaktionsgemisches erhält man ein erstes Rohprodukt, das in der Regel überwiegend aus Diarylcarbonat und/oder aromatischer Hydroxyverbindung besteht, und das noch gewisse Mengen an Chlorameisensäureester enthält, die in der Regel <50 Gew.-%, bevorzugt <30 Gew.-%, ganz besonders bevorzugt <15 Gew.-% betragen.

Dieses Gemisch kann man direkt der destillativen Aufarbeitung zuführen und in Ströme aus Chlorameisensäureester und Hydroxyverbindung, einem Diarylcarbonat und kleinen Mengen Hochsieder aufteilen. Zur Vereinfachung und ökonomischeren Durchführung der Destillation ist jedoch ein Reaktionsgemisch besser geeignet, das keinen oder nur kleine Mengen an Chlorameisensäureester enthält.

Daher leitet man vorteilhafterweise das nach der Entgasung erhaltene erste Rohprodukt in einen zweiten, heterogenen Katalysator enthaltenden Reaktor und setzt darin den noch vorhandenen Chlorameisensäureester mit im Gemisch noch befindlicher oder zugesetzter Hydroxyverbindung unter ähnlichen Bedingungen wie im ersten Reaktor um. Dabei kann der Druck in engeren Grenzen von 0,6 bis 6, vorzugsweise 0,8 bis 4 bar und die Temperatur etwas höher, nämlich von 120 bis 250°C, vorzugsweise 140 bis 240°C, besonders bevorzugt 160 bis 230°C liegen.

Die Belastung der Reaktoren, gemessen in Kilogramm Eduktgemisch pro Liter Katalysatorvolumen und Stunde hängt von der Reaktionstemperatur, der Aktivität der Katalysatoren und dem gewünschten Umsatz ab. Sie beträgt 0,01 bis 20, vorzugsweise 0,02 bis 10, besonders bevorzugt 0,05 bis 4, ganz besonders bevorzugt 0,1 bis 3 kg/l·h.

Auch das den zweiten Reaktor verlassende Gemisch wird entgast und der Abgasstrom ebenfalls in den Abgasnachreaktor geleitet. Das entgaste Gemisch, das nur noch geringe Mengen (<3, bevorzugt <2, besonders bevorzugt <1 Gew.-%) Chlorameisensäureester enthält, wird in einer ersten Destillationskolonne von überschüssiger Hydroxyverbindung und Chlorameisensäurester befreit, die als Kopfprodukt abgenommen und je nach Bedarf in den Reaktor, Nachreaktor oder in den Abgasnachreaktor geleitet und weiter umgesetzt werden.

Das am Boden dieser Kolonne ablaufende Gemisch wird in einer zweiten Kolonne in restliche Leichtsieder, die in den oberen Teil der ersten Kolonne zurückgeführt werden, reines Diarylcarbonat, das seitlich aus dem Dampfstrom dieser zweiten Kolonne entnommen wird, und in ein Gemisch aus Diarylcarbonat und Hochsieder, das die Kolonne als Sumpf verläßt, aufgetrennt.

Durch Einspeisung in einer dritten Kolonne wird zwecks Feinreinigung das Diphenylcarbonat, das als Kopfprodukt dieser dritten Kolonne entnommen wird, von Spuren Hochsieder (Sumpf) getrennt.

Die vereinigten Sümpfe aus der zweiten und dritten Kolonne trennt man in einer vierten Destillationsvorrichtung, die kontinuierlich oder diskontinuierlich betrieben wird, in einen die Hochsieder enthaltenden Sumpf und Diarylcarbonat auf, das in den unteren Teil der zweiten Kolonne geleitet und dort weiter gereinigt wird.

Die vergleichsweise geringen Mengen Sumpf von <3, vorzugsweise <2, besonders bevorzugt <1 % des Reaktionsproduktes werden zweckmäßig verbrannt oder zur Phenolharzherstellung eingesetzt.

### Beispiele

### Kontinuierliche Herstellung von Diphenylcarbonat durch Phosgenierung von Phenol in Gegenwart von γ-Aluminiumoxid unter Rückführung des Abgases in einen Nachreaktor

### Beispiel 1

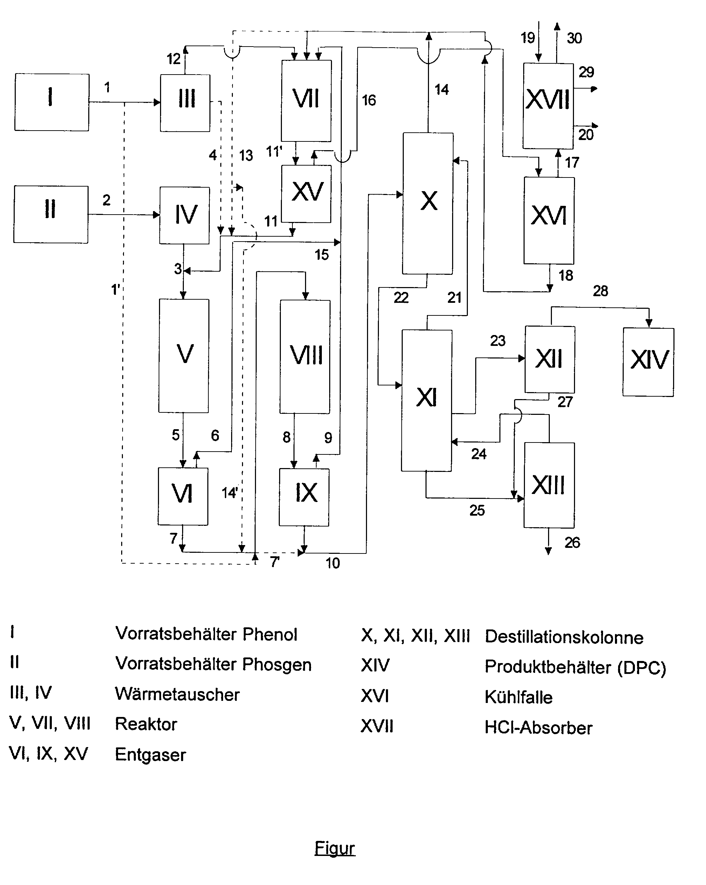

Die zur Durchführung des erfindungsgemäßen Verfahrens eingesetzte Apparatur und die auftretenden Stoffströme sind schematisch in der Figur wiedergegeben.

Zusammen mit aus der Destillationskolonne X am Kopf entnommenem Phenol 14 und aus der Kühlfalle XVI zurückgeführtem Phenol 18 dosiert man aus einem beheizten Behälter I über Wärmetauscher III (60°C) unter Normaldruck 28,0 Gew.-Teile/h Phenol 12 von oben in einen auf 170°C beheizten, mit 150 Vol.-Teilen γ-Aluminiumoxid gefüllten Abgasreaktor VII. In Gleichstrom damit wird der aus den Entgasungsapparaturen VI und IX stammende Abgasstrom 15 (Gewichtsverhältnis Phenol/Phosgen/Chlorwasserstoff/Kohlendioxid 2,9/8,9/87,6/0,6) in Reaktor VII eingespeist.

Das am Fuß des Reaktors austretende Produkt 11', das Phenol, Chlorameisensäurephenylester, Diphenylcarbonat und Nebenprodukte im Verhältnis 95,6/0,01/4,3/0,08 enthält, wird über Entgaser XV in Abgas 16 (Gewichtsverhältnis Phenol, Phosgen, Chlorwasserstoff und Kohlendioxid 3,7/1,3/94,4/0,6) und Sumpf 11 (Gewichtsverhältnis Phenol, Chlorameisensäurephenylester, Diphenylcarbonat und Nebenprodukte 95,6/0,01/4,4/0,08) aufgetrennt.

15,05 Gew.-Teile/h über Wärmetauscher IV (170°C) vorgeheiztes Phosgen 3 wird zusammen mit 11 in einem auf 170°C beheizten, mit 150 Vol.-Teilen γ-Aluminiumoxid gefüllten Reaktor V in Gleichstrom eingeleitet.

Das am Fuß des Reaktors austretende Produkt 5, das Phenol, Chlorameisensäurephenylester, Diphenylcarbonat und Nebenprodukte im Verhältnis 34,3/0,2/65,1/0,4 enthält, wird über Entgaser VI in Abgas 6 (Gewichtsverhältnis Phenol, Phosgen, Chlorwasserstoff und Kohlendioxid 1,5/9,0/88,9/0,6) und Sumpf 7 (Gewichtsverhältnis Phenol, Chlorameisensäurephenylester, Diphenylcarbonat und Nebenprodukte 34,0/0,2/65,4/0,4) aufgetrennt.

Der im Sumpf 7 vorhandene Chlorameisensäurephenylester wird durch Nachreaktion mit vorhandenem Phenol (eventuell nach Zugabe von zusätzlichem Phenol (1' oder 14') in einem Nachreaktor VIII, ebenfalls befüllt mit γ-Aluminiumoxid (150 Vol.-Teilen) bei 180°C zu Diphenylcarbonat umgesetzt.

Das am Reaktorfuß entnommene Produkt 8 (Gewichtsverhältnis Phenol, Diphenylcarbonat und Nebenprodukte 33,9/65,7/0,4) wird über Entgaser IX in Abgas 9 (Phenol/Chlorwasserstoff) und Sumpf 10 (Gewichtsverhältnis Phenol, Diphenylcarbonat und Nebenprodukte 33,7/65,9/0,4) aufgetrennt.

Abgasströme 6 und 9 werden vereinigt zu 15 und in Abgasnachreaktor VII in Gleichstrom mit Phenol (12, 14 und 18) geleitet, wie oben bereits erwähnt.

Das am Kopf des Entgasers XV austretende Abgas 16 wird einer Kühlfalle XVI zugeleitet, in der vorhandenes Phenol 18 (0,43Gew.-Teile/h) entfernt und in Abgasnachreaktor VII zurückgegeben wird.

Das aus der Kühlfalle XVI austretende Abgas 17 wird einer Chlorwasserstoffabsorptionsanlage XVII zugeführt.

Durch Einspeisung von 64,2 Gew.-Teilen/h einer 18 %igen Salzsäure 19 erhält man 75,1 Gew.-Teile/h einer 30 %igen Salzsäure 20, die der Elektrolyse zugeführt werden kann. Das aus der Elektrolyse gewonnene Chlor kann wieder für die Herstellung von Phosgen verwendet werden.

Spuren mitgeführtes Phenol können als Azeotrop mit Wasser 29 entfernt werden.

Zur Zersetzung noch in Spuren vorhandenen Phosgens im Abgas 30 wird eine Vernichtungsanlage (Aktivkohletürme mit Wasser) angeschlossen.

Sumpf 10 wird in eine erste Destillationskolonne X eingespeist und bei ca. 80°C/12 mm aufgetrennt in 16,1 Gew.-Teile/h Phenol und Sumpf 22 (Gewichtsverhältnis Phenol, Diphenylcarbonat und Nebenprodukte 0,3/99,1/0,6).

Sumpf 22 wird einer zweiten Destillationskolonne XI zugeleitet, in der über Kopf noch vorhandenes Phenol 21 (0,1 Gew.-Teile/h) entfernt und in den oberen Teil der ersten Kolonne X zurückgegeben wird.

Durch seitliche Ausspeisung aus dem Gasraum der zweiten Kolonne XI erhält man 31,7 Gew.-Teile/h Produkt 23 (Gewichtsverhältnis Diphenylcarbonat, Nebenprodukte 99,9/0,1), das in einer dritten Destillationskolonne XII in Kopfprodukt 28 (31,7 Gew.-Teile/h Diphenylcarbonat), entsprechend einer Selektivität von 99,6 %, bezogen auf Phenol, und Sumpf 27 aufgetrennt wird.

Das am Fuß der Destillationskolonne XI entnommene Produkt 25 (Gewichtsverhältnis Diphenylcarbonat/Nebenprodukte 91,1/8,9) wird zusammen mit 27 in einer vierten Destillationskolonne XIII bei 170°C/12mm in Kopfprodukt 24 (1,4 Gew.-Teile/h Diphenylcarbonat), das in den unteren Teil der zweiten Kolonne XI zurückgeführt wird, und Sumpf 26 (hochsiedende Nebenprodukte) aufgetrennt.

### Beispiel 2

Durchführung des erfindungsgemäßen Verfahrens wie in Beispiel 1 beschrieben, unter Einspeisung von 20,10 Gew.-Teilen/h Phenol 12 über Wärmetauscher III in Abgasreaktor VII und Einleitung von 10,69 Gew.-Teilen/h über Wärmetauscher IV vorgeheiztem Phosgen 3 in Reaktor V, führt zu einem phosgenfreien Abgasstrom 16, bei gleichbleibender Selektivität für Diphenylcarbonat.

Als weitere Variationen zu der beschriebenen Verfahrensweise sind in Abhängigkeit von den Edukt- und Produktzusammensetzungen, Katalysatorbelastungen und Temperatur zu nennen:
a) Dosierung von Phenol 4 direkt in Reaktor V statt über Abgasnachreaktor VII beim Anfahren.
b) Zugabe von Phenol (1' oder 14') als zusätzlicher Reaktionspartner für die Nachreaktion bei Vorliegen größerer Konzentrationen an Chlorameisensäurephenylester.
c) Fahrweise ohne Nachreaktion im Reaktor VIII, wobei dann noch vorhandener Chlorameisensäurephenylester in der ersten Destillationskolonne X als Leichtsieder 14 mit Phenol abdestilliert und in Abgasnachreaktor VII eingespeist wird, oder bei einer Fahrweise ohne Abgasnachreaktor in den ersten Reaktor V zurückgeführt wird (13).
d) Flüssige Phase und Phosgen werden im Gegenstrom durch Reaktor V geleitet. Dabei tritt Phosgen aus dem Vorratsbehälter II über Wärmetauscher IV von unten in den Reaktor V ein, die flüssige Phase von oben. Zur Abführung des gebildeten Abgases wird der obere Teil des Reaktors V zusätzlich mit Abgasnachreaktor VII verbunden.

## Patentansprüche

1. Verfahren zur kontinuierlichen Herstellung von Diarylcarbonaten durch Umsetzung von Phosgen mit aromatischen Hydroxyverbindungen in Gegenwart von heterogenen Katalysatoren, wobei man
1) ein Gemisch aus aromatischer Hydroxyverbindung und gegebenenfalls deren Chlorameisensäureester zusammen mit Phosgen in einen mit heterogenem Katalysator gefüllten Reaktor einleitet und darin so reagieren läßt, daß die Reaktionswärme durch Verdampfen der Edukte und Produkte abgeführt wird und die Reaktionstemperatur um maximal 50°C über die Eintrittstemperatur des Reaktionsgemisches steigt,
2) das den Reaktor verlassende Produkt entgast, das Abgas zusammen mit einem gerade schmelzflüssigen Strom der aromatischen Hydroxyverbindung, der gegebenenfalls etwas Chlorameisensäureester enthält, in einen mit heterogenem Katalysator gefüllten Abgasnachreaktor einleitet und darin so reagieren laßt, daß Phosgen und gegebenenfalls Chlorameisensäureester aus dem Abgasstrom entfernt werden,
3) das aus dem Reaktor entnommene und entgaste Reaktionsprodukt entweder direkt der Aufarbeitung zuführt oder aber in einen Nachreaktor einspeist, in dem restlicher Chlorameisensäureester über heterogenen Katalysator mit noch vorhandener oder zugespeister aromatischer Hydroxyverbindung weiter zu Diarylcarbonat umgesetzt wird,
4) das den Nachreaktor verlassende Produkt wiederum entgast, und dieses Abgas dem unter 2) genannten Abgasnachreaktor mit schmelzflüssiger aromatischer Hydroxyverbindung zuführt,
5) das entgaste Produkt aus dem Nachreaktor in eine Destillationskolonne einspeist, aromatische Hydroxyverbindung und gegebenenfalls noch vorhandene Chlorameisensäureester über Kopf abdestilliert und in den Abgasnachreaktor oder in den ersten Reaktor wieder einleitet,
6) den Sumpf dieser ersten Kolonne einer zweiten Destillationskolonne zuleitet, in ihr über Kopf gegebenenfalls noch vorhandene Spuren Leichtsieder aus dem Diarylcarbonat entfernt, die in den oberen Teil der ersten Kolonne zurückgegeben werden,
7) aus dem Gasraum der zweiten Kolonne Diarylcarbonat, gegebenenfalls mit Spuren Hochsieder, ausspeist,
dadurch gekennzeichnet, daß man
8) dieses Produkt einer dritten Kolonne zuführt und durch Abtrennung von Restmengen Hochsieder als Sumpf reines Diphenylcarbonat als Kopfprodukt erhalt,
9) die vereinigten Sümpfe der zweiten und dritten Kolonne einer vierten Destillationseinheit zuführt, über Kopf Diarylcarbonat abdestilliert, es in die zweite Kolonne zurückleitet und aus dem Sumpf der vierten Destillationseinheit die Hochsieder entnimmt.

## Claims

1. A process for the continuous production of diaryl carbonates by the reaction of phosgene with aromatic hydroxy compounds in the presence of heterogeneous catalysts.
1) wherein a mixture of aromatic hydroxy compounds, and optionally chloroformic esters thereof, together with phosgene, is led into a reactor packed with a heterogeneous catalyst and is reacted therein so that the heat of reaction is dissipated by the vaporisation of the starting materials and products and the temperature of reaction rises by a maximum of 50°C above the inlet temperature of the reaction mixture.
2) the product leaving the reactor is degassed, the off-gas, together with a stream of the aromatic hydroxy compound which is just molten and which possibly contains some esters of chloroformic acid, is led into an off-gas secondary reactor packed with a heterogeneous catalyst and is reacted therein so that phosgene and esters of chloroformic acid, if present, are removed from the off-gas stream,
3) the degassed reaction product with is removed from the reactor is either fed directly to a work-up stage or is fed into a secondary reactor in which residual esters of chloroformic acid are further reacted over a heterogeneous catalyst, with the aromatic hydroxy compound which is still present or which is fed in, to form diaryl carbonate.
4) the product leaving the secondary reactor is again degassed, and this off-gas is fed with molten aromatic hydroxy compound to the off-gas secondary reactor cited in 2),
5) the degassed product from the secondary reactor is fed into a distillation column, aromatic hydroxy compound and, if applicable, esters of chloroformic acid which are still present are distilled off overhead and are led again into the off-gas secondary reactor or into the first reactor,
6) the bottom product from this column is fed to a second distillation column in which any traces of light-boiling fractions which possibly remain are removed overhead from the diaryl carbonate and arc recycled to the upper part of the first column.
7) diaryl carbonate, possibly together with traces of high-boiling fractions. is taken off from the gas space of the second column,
characterised in that
8) this product is fed to a third column and pure diphenyl carbonate is obtained as a top product by the separation of residual amounts of high-boiling fractions as a bottom product.
9) the combined bottom products from the second and third columns are fed to a fourth distillation unit, diaryl carbonate is distilled off overhead and is recycled to the second column, and the high-boiling fractions are removed from the bottom of the fourth distillation unit.

## Revendications

1. Procédé pour la préparation continue de carbonates de diaryle par réaction du phosgène avec des composés hydroxylés aromatiques en présence de catalyseurs hétérogènes, dans lequel
1) on envoie un mélange du composé hydroxylé aromatique et le cas échéant de son ester chloroformique avec le phosgène dans un réacteur garni de catalyseur hétérogène ou on le laisse réagir dans des conditions telles que la chaleur de réaction est évacuée par vaporisation des composants de départ et des produits formés et que la température de réaction monte au maximum de 50°C au-dessus de la température d'entrée du mélange de réaction,
2) on dégaze le produit quittant le réacteur, on envoie les gaz résiduaires, avec un courant juste fondu du composé hydroxylé aromatique, contenant le cas échéant un peu d'ester chloroformique, dans un réacteur complémentaire des gaz résiduaires garni de catalyseur hétérogène ou on les laisse réagir dans des conditions telles que le phosgène et le cas échéant l'ester chloroformique sont éliminés du courant de gaz résiduaires,
3) on envoie le produit de réaction qui a quitté le réacteur et qui a été dégazé soit directement aux opérations d'isolement soit dans un réacteur complémentaire dans lequel les résidus d'ester chloroformique sont convertis en carbonate de diaryle sur un catalyseur hétérogène par le composé hydroxylé aromatique encore présent ou introduit,
4) on dégaze à nouveau le produit quittant le réacteur complémentaire et on envoie les gaz résiduaires au réacteur complémentaire des gaz résiduaires dont il a été question ci-dessus sous 2) avec le composé hydroxylé aromatique fondu,
5) on envoie le produit dégazé sortant du réacteur complémentaire à une colonne à distiller d'où l'on distille en tête le composé hydroxylé aromatique et le cas échéant l'ester chloroformique encore présent qu'on renvoie au réacteur complémentaire des gaz résiduaires ou au premier réacteur,
6) on envoie le produit de pied de cette première colonne à une deuxième colonne à distiller dans laquelle on sépare du carbonate de diaryle, en tête, les traces résiduelles éventuelles de fractions légères, qu'on renvoie à la partie supérieure de la première colonne,
7) de l'espace des gaz de la deuxième colonne, on évacue le carbonate de diaryle, le cas échéant avec des traces de fractions lourdes,
ce procédé se caractérisant en ce que
8) on envoie ce produit à une troisième colonne dans laquelle on obtient, par séparation des résidus de fractions lourdes en pied de colonne, le carbonate de diphényle pur en tête de colonne,
9) on envoie les produits de pieds de colonnes combinés de la deuxième et de la troisième colonne à une quatrième unité de distillation d'où l'on évacue en tête le carbonate de diaryle qu'on renvoie à la deuxième colonne, et on évacue de la quatrième unité de distillation les fractions lourdes en produit de pied.
